# EUROPEAN PATENT APPLICATION

(11) **EP 4 170 335 A1**
(43) Date of publication of application: **26.04.2023**
(21) Application number: 22198972.6
(22) Date of filing: 30.09.2022
(51) Int. Cl.: G01N 27/404, G01N 33/00

(54) **LOW CROSS-SENSITIVITY OF ELECTROCHEMICAL GAS DETECTING APPARATUSES**

(30) Priority: 21.10.2021 CN 202111225496
(71) Applicant: Honeywell International Inc., Charlotte, NC 28202 (US)
(72) Inventor: LIANG, Feng, Charlotte, 28202 (US); LV, Hongling, Charlotte, 28202 (US); GUAN, Pengjian, Charlotte, 28202 (US); LI, Wenjuan, Charlotte, 28202 (US); XIAO, Lei, Charlotte, 28202 (US); ZHOU, Yan, Charlotte, 28202 (US); HU, Yu, Charlotte, 28202 (US)
(74) Representative: Haseltine Lake Kempner LLP

(57) **Abstract**

Methods, apparatuses and systems for providing electrochemical gas detecting apparatuses are disclosed herein. The gas detecting apparatus may comprise a sensing component comprising: a housing configured to receive a sample gaseous substance comprising a target gaseous substance and an interferent gaseous substance; a reference electrode, disposed within the housing; a counter electrode disposed within the housing; and a sensing electrode disposed within the housing that is operatively coupled to a bias voltage circuit. The sensing electrode is configured to generate a current signal between the sensing electrode and the counter electrode indicative of a concentration level of the target gaseous substance in response to a first electrochemical reaction that occurs with respect to the target gaseous substance in an instance in which the sample gaseous substance is incident on the sensing electrode, the reference electrode is configured to maintain a potential of the sensing electrode at a fixed value, and the bias voltage circuit operates to modify a direction of a second electrochemical reaction that occurs with respect to the interferent gaseous substance.

## Description

### BACKGROUND

Gas detecting apparatuses (e.g., electrochemical detectors) may be utilized to detect and/or measure concentration levels of gaseous substances and/or compounds in gaseous substances, including, for example, organic compounds and inorganic compounds. Many gas detecting devices are plagued by technical challenges and limitations.

### BRIEF SUMMARY

Various embodiments described herein relate to methods, apparatuses, and systems for providing gas detecting apparatuses.

In accordance with various examples of the present disclosure, a gas detecting apparatus is provided. In some examples, the gas detecting apparatus comprises a sensing component, the sensing component comprising at least: a housing configured to receive a sample gaseous substance comprising a target gaseous substance and an interferent gaseous substance; a reference electrode disposed within the housing, a counter electrode disposed within the housing; and a sensing electrode disposed within the housing that is operatively coupled to a bias voltage circuit, wherein: the sensing electrode is configured to generate a current signal between the sensing electrode and the counter electrode indicative of a concentration level of the target gaseous substance in response to a first electrochemical reaction that occurs with respect to the target gaseous substance in an instance in which the sample gaseous substance is incident on the sensing electrode, the reference electrode is configured to maintain a potential of the sensing electrode at a fixed value, and the bias voltage circuit operates to modify a direction of a second electrochemical reaction that occurs with respect to the interferent gaseous substance.

In accordance with various examples of the present disclosure, a method is provided. In some examples, the method comprises: receiving, within a housing of a sensing component, a sample gaseous substance comprising a target gaseous substance and an interferent gaseous substance; generating, by a sensing electrode of the sensing component, a current signal between the sensing electrode and a counter electrode of the sensing component indicative of a concentration level of the target gaseous substance in response to a first electrochemical reaction that occurs with respect to the target gaseous substance in an instance in which the sample gaseous substance is incident on the sensing electrode, maintaining, by a reference electrode of the sensing component, a potential of the sensing electrode at a fixed value, and modifying, via a bias voltage circuit that is operatively coupled to the sensing electrode, a direction of a second electrochemical reaction that occurs with respect to the interferent gaseous substance.

The foregoing illustrative summary, as well as other exemplary objectives and/or advantages of the disclosure, and the manner in which the same are accomplished, are further explained in the following detailed description and its accompanying drawings.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 illustrates a schematic diagram depicting an example apparatus in accordance with various embodiments of the present disclosure;
FIG. 2 illustrates an example bias circuit in accordance with various embodiments of the present disclosure;
FIG. 3 illustrates a graph depicting example measurements in accordance with various embodiments of the present disclosure;
FIG. 4 illustrates a graph depicting example measurements in accordance with various embodiments of the present disclosure;
FIG. 5 illustrates a graph depicting example measurements in accordance with various embodiments of the present disclosure; and
FIG. 6 illustrates an example controller component in electronic communication with other element(s)/component(s) of an example gas detecting apparatus in accordance with various embodiments of the present disclosure.

### DETAILED DESCRIPTION OF THE INVENTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

The components illustrated in the figures represent components that may or may not be present in various embodiments of the present disclosure described herein such that embodiments may include fewer or more components than those shown in the figures while not departing from the scope of the present disclosure. Some components may be omitted from one or more figures or shown in dashed line for visibility of the underlying components.

The phrases "in an example embodiment," "some embodiments," "various embodiments," and the like generally mean that the particular feature, structure, or characteristic following the phrase may be included in at least one embodiment of the present disclosure, and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such components or features may be optionally included in some embodiments, or may be excluded.

The term "electronically coupled" or "in electronic communication with" in the present disclosure refer to two or more electrical elements (for example, but not limited to, an example processing circuitry, communication module, input/output module memory, humidity sensing component, cooling element, gas detection component) and/or electric circuit(s) being connected through wired means (for example but not limited to, conductive wires or traces) and/or wireless means (for example but not limited to, wireless network, electromagnetic field), such that data and/or information (for example, electronic indications, signals) may be transmitted to and/or received from the electrical elements and/or electric circuit(s) that are electronically coupled.

Various apparatuses (such as, but not limited to, a gas detecting apparatus (e.g., electrochemical detector)) may detect and/or measure a concentration level of one or more target gaseous substances and/or target compounds in a gaseous substance, in some examples, within a specified location or area in order to satisfy regulations and/or meet ambient air quality standards. Target gaseous substances/compounds may include volatile organic compounds (VOCs), toxic gases and the like.

In various examples, a gas detecting apparatus may comprise a sensing component that includes a sensing electrode that is configured to detect/measure a concentration level of a target gaseous substance (e.g., Sulfur Dioxide (SO₂)) in an environment. For example, a sample gaseous substance may be directed to flow into a sensing component of a gas detecting apparatus so that it makes contact with (e.g., passes through) a sensing electrode. Many such gas detecting apparatuses (e.g., electrochemical detectors) for detecting the presence of one or more target gaseous substances and/or compounds as described above may present many technical challenges and limitations.

In some embodiments, in monitoring for the presence of a target gaseous substance, other gaseous substances may be present that may react (e.g., generate an electrochemical reaction) with the same sensing electrode. Accordingly, in some examples, a gas detecting apparatus (e.g., electrochemical detector) comprising a sensing electrode configured to detect/measure a concentration level of a target gaseous substance may also react to the presence of another gaseous substance (e.g., an interferent gaseous substance). By way of example, a sensing electrode of a gas detecting apparatus/electrochemical sensor for detecting SO₂ may also detect/react with a common interferent gaseous substance such as a nitrogen oxide (e.g., Nitrogen Dioxide (NO₂)). In particular, SO₂ may undergo an oxidizing reaction in response to making contact with a sensing electrode and thus generate a positive signal indicative of the presence of and/or concentration level of SO₂. The below formula describes an example of an oxidizing reaction for SO₂:

*SO*₂ + 2*H*₂*O* → *H*₂*SO*₄ + 2*H*⁺ + 2*e*⁻

However, in some examples, the interferent gaseous substance NO₂ may undergo a reducing reaction in response to making contact with the same sensing electrode and thus generate a negative signal. The below formula describes an example of a reducing reaction for NO₂:

*NO*₂ + 2*H*⁺ + 2*e*⁻ → NO + *H*₂*O*

As a result of the negative signal generated in response to the presence of NO₂, the output of an example gas detection apparatus that is configured to detect SO₂ may generate an inaccurate reading, e.g., by summing the positive signal generated by SO₂ and the negative signal generated by NO₂ to output an SO₂ reading that is different from (e.g., lower than) the actual concentration level of SO₂ in a sample gaseous substance. This may result in false negative outputs and/or inaccurate measurement of a concentration level of a target gaseous substance being generated by the example gas detector in various applications.

In some examples, in order to account for cross-sensitivity of an interferent gaseous substance, a gas detector may be configured to measure a concentration level of an interferent gaseous substance (e.g., a nitrogen oxide such as NO₂) in addition to detecting a concentration level of a target gaseous substance (e.g., SO₂). Then, the gas detector may be configured to compensate for the detected interferent gaseous substance (e.g., NO₂) in the target gaseous substance reading. However, the above example requires that a highly accurate reading is captured with respect to the interferent gaseous substance (e.g., NO₂) in order to accurately determine a target gaseous substance reading (e.g., SO₂). Accordingly, if the interferent gaseous substance reading (e.g., NO₂) is inaccurate, then the target gaseous substance reading (e.g., SO₂) will also be inaccurate.

In accordance with various embodiments of the present disclosure, example methods, apparatuses and systems are provided.

In some examples, a gas detecting apparatus is provided. The gas detecting apparatus may comprise a sensing component. The sensing component may comprise at least a housing configured to receive a sample gaseous substance comprising a target gaseous substance and an interferent gaseous substance, a reference electrode disposed within the housing, a counter electrode is disposed within the housing and a sensing electrode disposed within the housing that is operatively coupled to a bias voltage circuit. In some examples, the sensing electrode is configured to generate a current signal between the sensing electrode and the counter electrode indicative of a concentration level of the target gaseous substance in response to a first electrochemical reaction that occurs with respect to the target gaseous substance in an instance in which the sample gaseous substance is incident on the sensing electrode. In some examples, the reference electrode is configured to maintain a potential of the sensing electrode at a fixed value. In some examples, the bias voltage circuit operates to modify a direction of a second electrochemical reaction that occurs with respect to the interferent gaseous substance. In some examples, the second electrochemical reaction occurs responsive to an above-threshold bias voltage provided to the sensing electrode via the bias voltage circuit. In some examples, the bias voltage circuit has an operating range between 90-150 mV. In some examples, the target gaseous substance comprises Sulfur Dioxide (SO₂). In some examples, the interferent gaseous substance comprises NO₂. In some examples, the housing contains an electrolyte material that comprises an agent. In some examples, the agent comprises a nitrate salt or ion. In some examples, the gas detecting apparatus further comprises a controller component in electronic communication with the sensing component. In some examples, the controller component is configured to receive an indication of the current signal, and determine a concentration level of the target gaseous substance based at least in part on the indication of the current signal. In some examples, the gas detecting apparatus comprises an electrochemical gas sensor.

In some examples, a method is provided. The method may comprise receiving, within a housing of a sensing component, a sample gaseous substance comprising a target gaseous substance and an interferent gaseous substance. In some examples, the method may comprise generating, by a sensing electrode of the sensing component, a current signal between the sensing electrode and a counter electrode of the sensing component indicative of a concentration level of the target gaseous substance in response to a first electrochemical reaction that occurs with respect to the target gaseous substance in an instance in which the sample gaseous substance is incident on the sensing electrode. In some examples, the method may comprise maintaining, by a reference electrode of the sensing component, a potential of the sensing electrode at a fixed value. In some examples, the method may comprise, modifying, via a bias voltage circuit that is operatively coupled to the sensing electrode, a direction of a second electrochemical reaction that occurs with respect to the interferent gaseous substance. In some examples, the second electrochemical reaction occurs responsive to an above-threshold bias voltage provided to the sensing electrode via the bias voltage circuit. In some examples, the bias voltage circuit has an operating range between 90-150 mV. In some examples, the target gaseous substance comprises Sulfur Dioxide (SO₂). In some examples, the interferent gaseous substance comprises Nitrogen Dioxide (NO₂). In some examples, the housing contains an electrolyte material that comprises an agent. In some examples, the agent comprises a nitrate salt. In some examples, the method further comprises receiving, by a controller component in electronic communication with the sensing component, a current signal indication. In some examples, the method further comprises, determining, by the controller component, a concentration level of the target gaseous substance based at least in part on the current signal indication. In some examples, the sensing component is a portion of a gas detecting apparatus or electrochemical gas sensor.

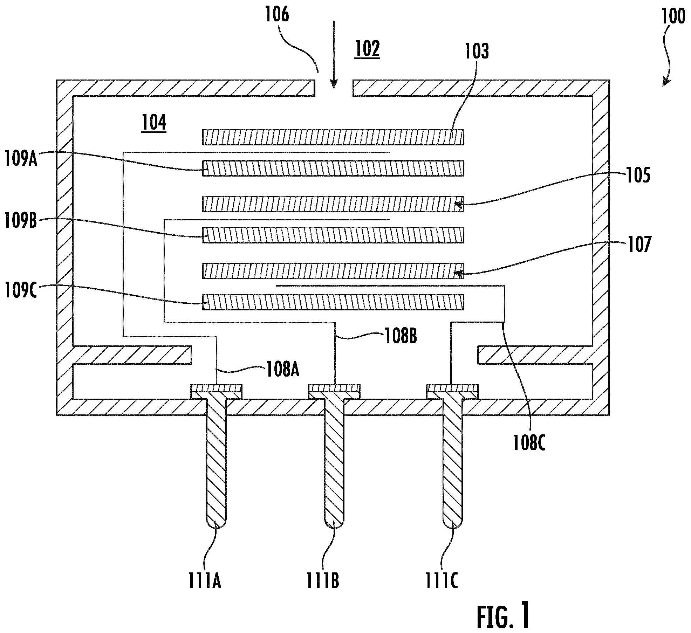

Referring now to FIG. 1, an example schematic diagram depicting a side section view of at least a portion of a sensing component 100 in accordance with some embodiments of the present disclosure is provided. In various embodiments, the sensing component 100 may be a portion of a gas detecting apparatus such as a electrochemical sensor. In various examples, as depicted, the sensing component 100 comprises a housing defining a cavity 104 or reservoir configured to contain an electrolyte solution. The example electrolyte may comprise an aqueous acidic electrolyte such as sulfuric acid, phosphoric acid, or a neutral ionic solution such as a salt solution (e.g., a lithium salt such as lithium chloride), combinations thereof, and/or the like. In some embodiments, the electrolyte may be in the form of a solid polymer electrolyte which comprises an ionic exchange membrane. In some embodiments, the electrolyte can be in the form of a free liquid, disposed in a matrix or slurry such as glass fibers, or disposed in the form of a semi-solid or solid gel.

As depicted in FIG. 1, the sensing component 100 comprises a plurality of elements. In particular, as shown, the sensing component 100 comprises a sensing electrode 103, a reference electrode 105, a counter electrode 107, and a plurality of separators 109A, 109B and 109C disposed within the cavity 104 defined by the housing of the sensing component 100. In various examples, the housing may comprise a polymeric material, a metal, or a ceramic. In some examples, the housing may comprise acrylonitrile butadiene styrene (ABS), polyphenylene oxide (PPO), polystyrene (PS), polypropylene (PP), polyethylene (PE) (e.g., high density polyethylene (HDPE)), polyphenylene ether (PPE), combinations thereof, and/or the like.

As depicted in FIG. 1, the sensing electrode 103 is disposed adjacent to an aperture 106 (e.g., gas diffusion barrier) on a top portion of the sensing component 100. The reference electrode 105 is disposed within a central portion of the sensing component 100 beneath the sensing electrode 103 and separated therefrom by a first separator 109A. As further depicted, the counter electrode 107 is disposed within a bottom portion of the sensing component 100 beneath the reference electrode 105 and separated therefrom by a second separator 109B. Additionally, as depicted, the counter electrode 107 is positioned adjacent a first sensor pin 111A, a second sensor pin 111B and a third sensor pin 111C which each protrude from a bottom surface of the sensing component 100. As further illustrated, a first end of a first current collector 108A is positioned between the sensing electrode 103 and the first separator 109A and a second end of the first current collector 108A is connected to the first sensor pin 111A. Similarly, a first end of a second current collector 108B is positioned between the reference electrode 105 and the second separator 109B and a second end of the second current collector 108B is connected to the second sensor pin 111B. As further depicted, a first end of a third current collector 108C is positioned between the counter electrode 107 and the third separator 109C and a second end of the third current collector 108C is connected to the third sensor pin 111C.

In various examples, the flow of electrons creates an electric current that is proportional to a concentration level of a target gaseous substance in the sample gaseous substance 102 that can be measured utilizing the first sensor pin 111A, the second sensor pin 111B and the third sensor pin 111C. As noted above, the sensing component 100 may be a portion of a gas detecting apparatus (e.g., electrochemical sensor). The gas detecting apparatus may be configured to further detect and/or amplify the current generated by the sensing component 100. Additionally, a controller component of the gas detecting apparatus may be configured to interpret characteristics of the current generated by the sensing component such that a concentration level of the target substance can be provided (e.g., in percent volume, parts per million (PPM), parts per billion (PPB), or the like). Additionally, the controller component may calculate and store a detected concentration level in memory.

In various embodiments, the example sensing component 100 is configured to detect and/or measure a concentration level of a target gaseous substance in a sample gaseous substance 102 (e.g., air sample). In various examples, the example sensing component 100 may be configured to detect and/or measure a concentration level of a particular gaseous substance such as SO₂. As the sample gaseous substance 102 enters the sensing component 100 via the aperture 106 to be incident on the sensing electrode 103, an electrochemical reaction occurs. In various examples, the electrochemical reaction may be an oxidation or reduction depending on the type of sample gaseous substance 102. For example, carbon monoxide may be oxidized to carbon dioxide, or oxygen may be reduced to water. In another example, SO₂ may be oxidized according to the above-noted formula. An example oxidation reaction causes a flow of electrons from the sensing electrode 103 to the counter electrode 107, generating a measurable positive current signal. Conversely, an example reduction reaction causes a flow of electrons from the counter electrode 107 to the sensing electrode 103, generating a measurable negative current signal.

In some embodiments, in order to reduce the cross sensitivity of a target gaseous substance (e.g., SO₂) and an interferent gaseous substance such as Nitrogen Dioxide (e.g., NO₂), a bias voltage circuit is operatively coupled to the sensing electrode 103. In some examples, the operating range of the bias voltage circuit is between 90-150 mV. In the example of a sensing component 100 for detecting and/or measuring a concentration level of SO₂, the bias voltage provided by the bias voltage circuit may enhance a first electrochemical reaction with respect to a target gaseous substance (e.g., the oxidizing reaction with respect to SO₂) and modify a direction of a second electrochemical reaction with respect to an interferent gaseous substance (e.g., reduce the reducing reaction with respect to NO₂) thereby providing more accurate measurements/readings.

By way of example, as a result of providing/applying an above-threshold bias voltage to the sensing electrode 103 (e.g., in some examples, between 90-150 mV), the interferent gaseous substance, NO₂, will be oxidized on the sensing electrode 103 instead of being reduced and will generate a positive signal instead of a negative signal. The below formula describes an example of an oxidizing reaction for NO₂ as a result of providing/applying a bias voltage to the sensing electrode 103:

*NO*₂ + *H*₂*O* → H*NO*₃ + *H*⁺ + *e*⁻

Accordingly, by applying/providing a bias voltage to the sensing electrode 103, the electrochemical reaction caused by NO₂ is modified/converted in a different direction (e.g., oxidized instead of reduced).

As depicted in FIG. 1, the sensing component 100 comprises a reference electrode 105. In various examples, the reference electrode 105 operates to stabilize the potential of/provide a reference for the potential of the sensing electrode 103 during the electrochemical reaction(s) caused by a sample gaseous substance 102. For example, the reference electrode 105 may operate to maintain (e.g., hold) the potential of the sensing electrode 103 at a fixed value. In some examples, the reference electrode 105 may provide a reference for the detected current and potential between the sensing electrode 103 and the counter electrode 107.

As noted herein, a sample gaseous substance 102 (such as an air sample) may flow into an aperture 106 through at least a portion of the example sensing component 100 such that the sample gaseous substance (e.g., air sample) flows through the sensing component 100 and makes contact with the sensing electrode 103. As depicted, the aperture 106 defines an opening on a top surface of the sensing component 100. However, the aperture 106 may define an opening on any other surface of the sensing component 100. By way of example, the aperture 106 may have a diameter between 150µm and 5mm. In some embodiments, the sensing component 100 may comprise a plurality of apertures through which a sample gaseous substance 102 may enter and exit the sensing component 100 (e.g., a gas inlet and a gas outlet). In some embodiments, the sample gaseous substance 102 may exit the sensing component 100 through the aperture 106. In some embodiments, the aperture 106 may comprise a gas diffusion barrier to restrict and/or direct the flow of gaseous substances (e.g., to the sensing electrode 103). The example diffusion barrier can be created by forming the aperture 106 as a capillary and/or a film or membrane can be used to control the mass flow rate through the aperture 106.

In various embodiments, the cavity 104 defined by the sensing component 100 which contains the above-noted elements (e.g., the sensing electrode 103, the reference electrode 105, the counter electrode 107) may comprise an electrolyte (e.g., a liquid electrolyte). The sensing electrode 103, the reference electrode 105, the counter electrode 107 may be in electrical contact with one another via the electrolyte within the cavity 104 of the sensing component 100. As noted above, the cavity of the 104 comprises a first separator 109A, a second separator 109B and a third separator 109C. Each of the first separator 109A, the second separator 109B and the third separator 109C may be or comprise a porous structure that operates to retain the electrolyte in contact with the sensing electrode 103, the counter electrode 107 and the reference electrode 105. In some embodiments, each of the first separator 109A, the second separator 109B and the third separator 109C may comprise a nonwoven porous material (e.g., a porous felt member), a woven porous material, a porous polymer (e.g., an open cell foam, a solid porous plastic) or the like. In general, each of the first separator 109A, the second separator 109B and the third separator 109C may be chemically inert with respect to the electrolyte and the sensing electrode 103, the reference electrode 105 and the counter electrode 107. In some embodiments, each of the first separator 109A, the second separator 109B and the third separator 109C may comprise various materials including, but not limited to, glass (e.g., a glass mat), polymer (plastic discs), ceramic, or the like. Each of the first separator 109A, the second separator 109B and the third separator 109C may operate as a wick for the retention and transportation of the electrolyte within the cavity of the sensing component 100 while providing electrical insulation to prevent shorting due to direct contact between any two electrodes.

In the example of a sensing component 100 for detecting/measuring a concentration level of SO₂, as discussed below, the electrolyte may comprise an agent (e.g., nitrate salt/ion or NO₃⁻) that further enhances the modification/change in direction of reactivity with respect an interferent gaseous substance (e.g., NO₂). For example, the nitrate salt may further reduce the movement of the electrochemical reaction balance with respect to NO₂ in thus lead to generating a current signal that is negligible or close to 0. Thus, in some embodiments of the present disclosure, cross-interference of NO₂ can be significantly reduced/eliminated.

While FIG. 1 provides an example of a sensing component 100, it is noted that the scope of the present disclosure is not limited to the example shown in FIG. 1. In some examples, an example sensing component 100 may comprise one or more additional and/or alternative elements, and/or may be structured/positioned differently than those illustrated in FIG. 1. For example, a sensing component 100 in accordance with the present disclosure may comprise two sensing electrodes.

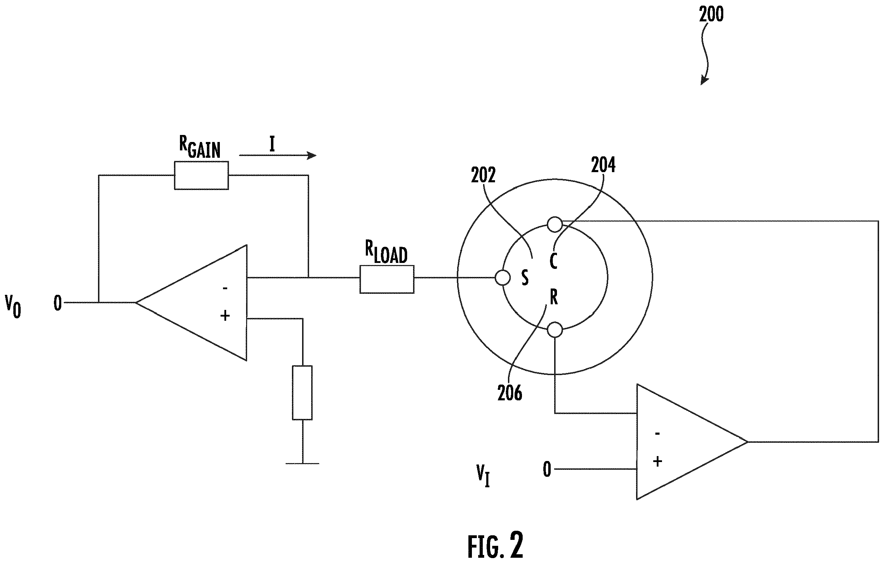

Referring now to FIG. 2, an example schematic diagram depicting a bias voltage circuit 200 in accordance with some embodiments of the present disclosure is provided. In various examples, as depicted, the bias voltage circuit comprises a potentiostat circuit that is configured to maintain correct bias potential(s) between a sensing electrode 202, a counter electrode 204 and a reference electrode 206. As noted above, in some examples, the operating range of the bias voltage circuit is between 90-150 mV. In various embodiments, a potential may be applied to the sensing electrode 202, the counter electrode 204 and/or the reference electrode 206 to maintain correct bias potential(s) therebetween. In some embodiments, the interferent gaseous substance NO₂ will be oxidized on the sensing electrode 202 when an above-threshold bias voltage (e.g., between 90-150 mV) is applied thereto.

While FIG. 2 provides an example of a bias voltage circuit 200, it is noted that the scope of the present disclosure is not limited to the example shown in FIG. 2. In some examples, an example bias voltage circuit 200 may comprise one or more additional and/or alternative elements, and/or may be structured/positioned differently than those illustrated in FIG. 2.

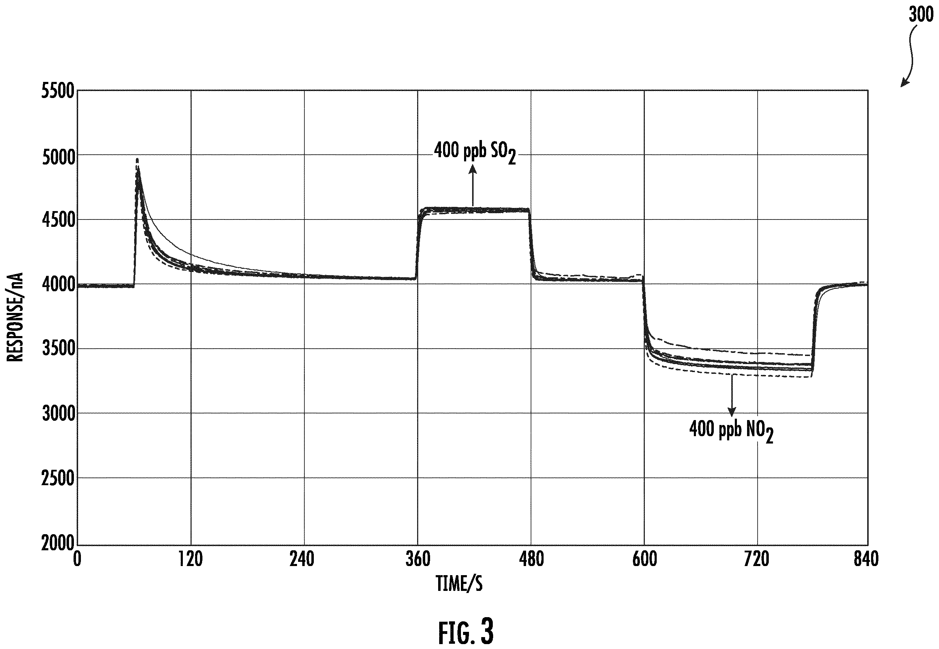

Referring now to FIG. 3 an example graph 300 depicting example measurements by a conventional sensing electrode is provided.

As depicted in FIG 3, the x-axis represents a plurality of instances in time. As depicted, the y-axis represents a detected current signal by a conventional sensing electrode measured in nano amperes (nA) associated with a sample gaseous substance comprising a target gaseous substance, 400 ppb of SO₂, and an interferent gaseous substance, 400 ppb of NO₂.

As illustrated in FIG. 3, responsive to the sample gaseous substance comprising SO₂ and NO₂ making contact with the sensing electrode, the target gaseous substance, SO₂ is oxidized to produce a positive current signal that is detected by the conventional sensing electrode (as depicted, approximately between 360 and 480 along the x-axis). Additionally, the interferent gaseous substance, NO₂ is reduced to produce a negative current signal that is also detected by the conventional sensing electrode (as depicted, approximately between 600 and 800 along the x-axis).

Accordingly, FIG. 3 demonstrates that the conventional sensing electrode will detect both SO₂ and NO₂. Thus it should be understood that the conventional sensing electrode may generate inaccurate measurements with respect to SO₂ in an instance in which an example gaseous substance comprises both SO₂ and the interferent gaseous substance NO₂ which in turn affects the accuracy of measurements that are outputted by an example gas detecting apparatus (e.g., electrochemical detector) associated therewith.

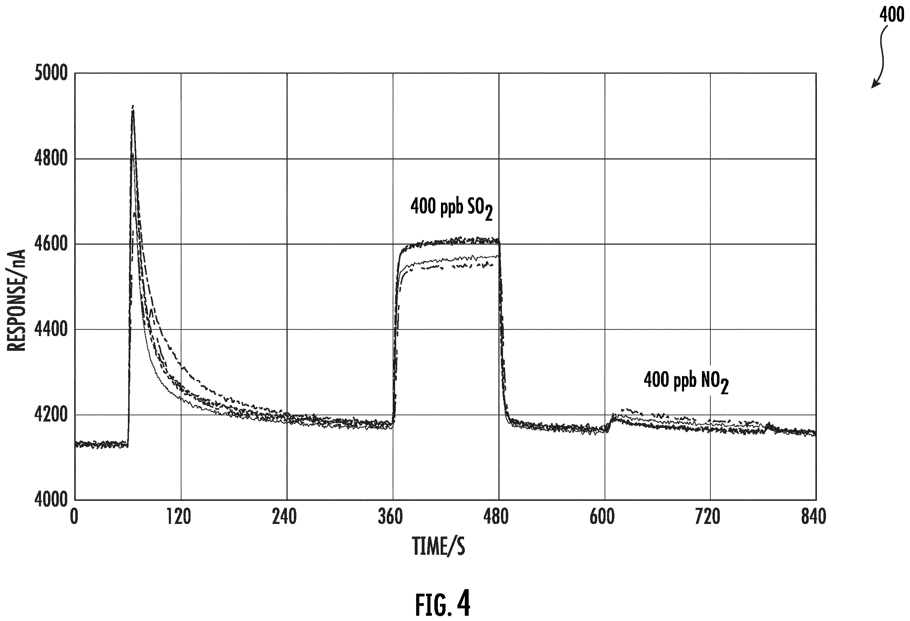

Referring now to FIG. 4, an example graph 400 depicting example measurements by a sensing electrode operatively coupled to a bias voltage circuit in accordance with various embodiments of the present disclosure are provided. The sensing electrode may be similar or identical to the sensing electrode 202 that is operatively coupled to the bias voltage circuit 200 discussed above in relation to FIG. 2. The sensing electrode may be a portion of a sensing component and/or gas detecting apparatus (e.g., electrochemical detector).

As depicted in FIG 4, the x-axis represents a plurality of instances in time. As depicted, the y-axis represents a detected current signal by the sensing electrode measured in nA associated with a sample gaseous substance comprising a target gaseous substance, 400 ppb of SO2, and an interferent gaseous substance, 400 ppb of NO₂. As illustrated in FIG. 4, responsive to the sample gaseous substance comprising SO2 and NO₂ making contact with the sensing electrode, the target gaseous substance, SO₂, is oxidized to produce a positive current signal that is detected by the sensing electrode (as depicted, approximately between 360 and 480 along the x-axis). However, in contrast with the example described above in relation to the conventional sensing electrode, the interferent gaseous substance, NO₂ is oxidized to produce a small positive current signal that is also detected by the sensing electrode (as depicted, approximately between 600 and 800 along the x-axis).

Accordingly, FIG. 4 demonstrates that the sensing electrode that is operatively coupled to a bias voltage circuit will accurately detect a positive current signal associated with SO₂. Additionally, as a result in modification of a direction of the electrochemical reaction associated with the interferent gaseous substance NO₂, only a small positive current signal associated with NO₂ will be detected by the sensing electrode. Thus, it should be understood that a sensing component incorporating the biased sensing electrode in accordance with the present disclosure will generate more accurate measurements with respect to SO₂, when an example gaseous substance comprises both SO₂ and NO₂ which in turn improves the accuracy of measurements that are outputted by an example gas detecting apparatus (e.g., electrochemical detector) associated therewith.

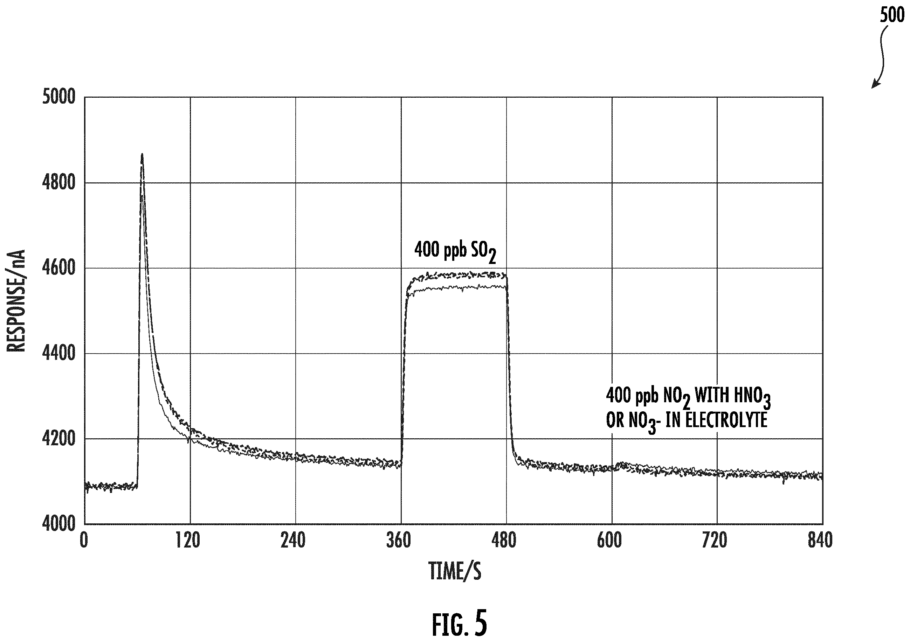

Referring now to FIG. 5, an example graph 500 depicting example measurements by a sensing electrode operatively coupled to a bias voltage circuit and in which the electrolyte comprises an agent (e.g., nitrate salt or NO₃⁻) in accordance with various embodiments of the present disclosure is provided. The sensing electrode may be similar or identical to the sensing electrode 202 that is operatively coupled to the bias voltage circuit 200 discussed above in relation to FIG. 2. The sensing electrode may be a portion of a sensing component and/or gas detecting apparatus (e.g., electrochemical detector).

As depicted in FIG 5, the x-axis represents a plurality of instances in time. As depicted, the y-axis represents a detected current signal by the sensing electrode measured in nA associated with a sample gaseous substance comprising a target gaseous substance, 400 ppb of SO₂, and an interferent gaseous substance, 400 ppb of NO₂. As illustrated in FIG. 4, responsive to the sample gaseous substance comprising SO₂ and NO₂ making contact with the sensing electrode, the target gaseous substance, SO₂, is oxidized to produce a positive current signal that is detected by the sensing electrode (as depicted, approximately between 360 and 480 along the x-axis). However, the interferent gaseous substance, NO₂ is also oxidized and as a result of the NO₃⁻ in the electrolyte produces a current signal that is close to 0 (as depicted, approximately between 600 and 800 along the x-axis).

Accordingly, FIG. 5 demonstrates that the sensing electrode that is operatively coupled to a bias voltage circuit that also comprises an electrolyte comprising NO₃⁻ will accurately detect a positive current signal associated with SO₂. Additionally, the current signal associated with the interferent gaseous substance NO₂ will be negligible or close to 0. Thus, it should be understood that a sensing component incorporating the sensing electrode in accordance with the present disclosure will generate more accurate measurements with respect to SO₂, when an example gaseous substance comprises both SO₂ and NO₂ and the use of NO₃⁻ will further improve/enhance the accuracy of measurements that are outputted by an example gas detecting apparatus (e.g., electrochemical detector) associated therewith.

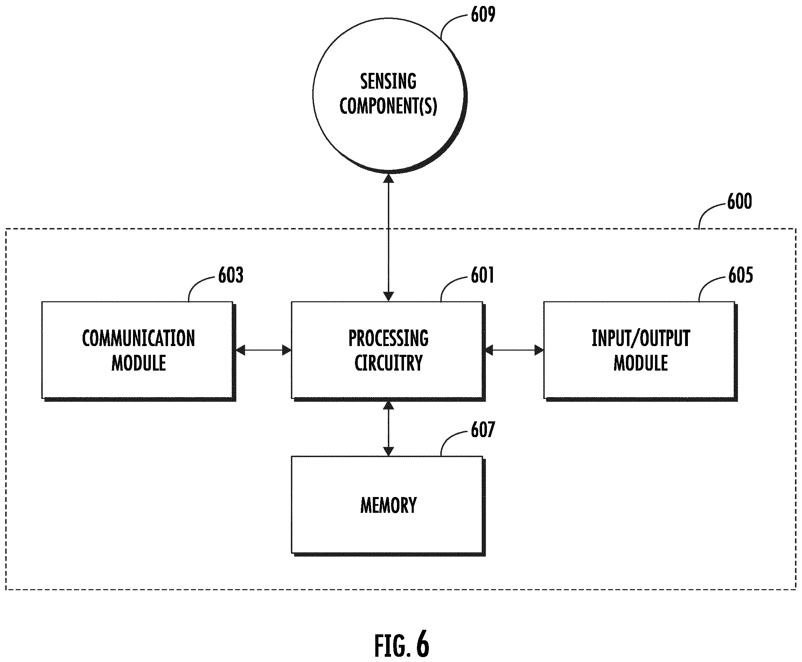

Referring now to FIG. 6, a schematic diagram depicting an example controller component 600 of an example gas detecting apparatus in electronic communication with various other components in accordance with various embodiments of the present disclosure. As shown, the controller component 600 comprises processing circuitry 601, a communication module 603, input/output module 605, a memory 607 and/or other components configured to perform various operations, procedures, functions or the like described herein.

As shown, the controller component 600 (such as the processing circuitry 601, communication module 603, input/output module 605 and memory 607) is electrically coupled to and/or in electronic communication with a sensing component 609 (e.g., comprising a gas detecting element). The sensing component 609 may be similar to the sensing component 100 described above in connection with FIG. 1. As depicted, the sensing component 609 may exchange (e.g., transmit and receive) data with the processing circuitry 601 of the controller component 600.

The processing circuitry 601 may be implemented as, for example, various devices comprising one or a plurality of microprocessors with accompanying digital signal processors; one or a plurality of processors without accompanying digital signal processors; one or a plurality of coprocessors; one or a plurality of multi-core processors; one or a plurality of controllers; processing circuits; one or a plurality of computers; and various other processing elements (including integrated circuits, such as ASICs or FPGAs, or a certain combination thereof). In some embodiments, the processing circuitry 601 may comprise one or more processors. In one exemplary embodiment, the processing circuitry 601 is configured to execute instructions stored in the memory 607 or otherwise accessible by the processing circuitry 601. When executed by the processing circuitry 601, these instructions may enable the controller component 600 to execute one or a plurality of the functions as described herein. No matter whether it is configured by hardware, firmware/software methods, or a combination thereof, the processing circuitry 601 may comprise entities capable of executing operations according to the embodiments of the present invention when correspondingly configured. Therefore, for example, when the processing circuitry 601 is implemented as an ASIC, an FPGA, or the like, the processing circuitry 601 may comprise specially configured hardware for implementing one or a plurality of operations described herein. Alternatively, as another example, when the processing circuitry 601 is implemented as an actuator of instructions (such as those that may be stored in the memory 607), the instructions may specifically configure the processing circuitry 601 to execute one or a plurality of algorithms and operations described herein, such as those discussed with reference to FIG. 5.

The memory 607 may comprise, for example, a volatile memory, a non-volatile memory, or a certain combination thereof. Although illustrated as a single memory in FIG. 6, the memory 607 may comprise a plurality of memory components. In various embodiments, the memory 607 may comprise, for example, a hard disk drive, a random access memory, a cache memory, a flash memory, a Compact Disc Read-Only Memory (CD-ROM), a Digital Versatile Disk Read-Only Memory (DVD-ROM), an optical disk, a circuit configured to store information, or a certain combination thereof. The memory 607 may be configured to store information, data, application programs, instructions, and etc., so that the controller component 600 can execute various functions according to the embodiments of the present disclosure. For example, in at least some embodiments, the memory 607 is configured to cache input data for processing by the processing circuitry 601. Additionally or alternatively, in at least some embodiments, the memory 607 is configured to store program instructions for execution by the processing circuitry 601. The memory 607 may store information in the form of static and/or dynamic information. When the functions are executed, the stored information may be stored and/or used by the controller component 600.

The communication module 603 may be implemented as any apparatus included in a circuit, hardware, a computer program product or a combination thereof, which is configured to receive and/or transmit data from/to another component or apparatus. The computer program product comprises computer-readable program instructions stored on a computer-readable medium (for example, the memory 607) and executed by a controller component 600 (for example, the processing circuitry 601). In some embodiments, the communication module 603 (as with other components discussed herein) may be at least partially implemented as the processing circuitry 601 or otherwise controlled by the processing circuitry 601. In this regard, the communication module 603 may communicate with the processing circuitry 601, for example, through a bus. The communication module 603 may comprise, for example, antennas, transmitters, receivers, transceivers, network interface cards and/or supporting hardware and/or firmware/software, and is used for establishing communication with another apparatus. The communication module 603 may be configured to receive and/or transmit any data that may be stored by the memory 607 by using any protocol that can be used for communication between apparatuses. The communication module 603 may additionally or alternatively communicate with the memory 607, the input/output module 605 and/or any other component of the controller component 600, for example, through a bus.

In some embodiments, the controller component 600 may comprise an input/output module 605. The input/output module 605 may communicate with the processing circuitry 601 to receive instructions input by the user and/or to provide audible, visual, mechanical or other outputs to the user. Therefore, the input/output module 605 may comprise supporting devices, such as a keyboard, a mouse, a display, a touch screen display, and/or other input/output mechanisms. Alternatively, at least some aspects of the input/output module 605 may be implemented on a device used by the user to communicate with the controller component 600. The input/output module 605 may communicate with the memory 607, the communication module 603 and/or any other component, for example, through a bus. One or a plurality of input/output modules and/or other components may be included in the controller component 600.

For example, the sensing component 609 may be similar to sensing component 100 described above with regard to FIG. 1. For example, sensing component 609 may generate measurements indicating a concentration level of one or more target gaseous substance in a sample gaseous substance and transmit a concentration level indication to the processing circuitry 601.

Many modifications and other embodiments of the present disclosure set forth herein will come to mind to one skilled in the art to which these embodiments pertain having the benefit of the teachings presented in the foregoing descriptions and the associated drawings. Therefore, it is to be understood that the disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A gas detecting apparatus comprising a sensing component, the sensing component comprising at least:
a housing configured to receive a sample gaseous substance comprising a target gaseous substance and an interferent gaseous substance;
a reference electrode disposed within the housing;
a counter electrode disposed within the housing; and
a sensing electrode disposed within the housing that is operatively coupled to a bias voltage circuit, wherein:
the sensing electrode is configured to generate a current signal between the sensing electrode and the counter electrode indicative of a concentration level of the target gaseous substance in response to a first electrochemical reaction that occurs with respect to the target gaseous substance in an instance in which the sample gaseous substance is incident on the sensing electrode,
the reference electrode is configured to maintain a potential of the sensing electrode at a fixed value, and
the bias voltage circuit operates to modify a direction of a second electrochemical reaction that occurs with respect to the interferent gaseous substance.

2. The gas detecting apparatus of claim 1, wherein the second electrochemical reaction occurs responsive to an above-threshold bias voltage provided to the sensing electrode via the bias voltage circuit.

3. The gas detecting apparatus of claim 2, wherein the bias voltage circuit has an operating range between 90-150 mV.

4. The gas detecting apparatus of claim 1, wherein the target gaseous substance comprises Sulfur Dioxide (SO₂).

5. The gas detecting apparatus of claim 1, wherein the interferent gaseous substance comprises Nitrogen Dioxide (NO₂).

6. The gas detecting apparatus of claim 1, wherein the housing contains an electrolyte material that comprises an agent.

7. The gas detecting apparatus of claim 6, wherein the agent comprises a nitrate salt.

8. The gas detecting apparatus of claim 1, further comprising a controller component in electronic communication with the sensing component.

9. The gas detecting apparatus of claim 8, wherein the controller component is configured to:
receive an indication of the current signal; and
determine a concentration level of the target gaseous substance based at least in part on the indication of the current signal.

10. The gas detecting apparatus of claim 1, wherein the gas detecting apparatus comprises an electrochemical gas sensor.

11. A method comprising:
receiving, within a housing of a sensing component, a sample gaseous substance comprising a target gaseous substance and an interferent gaseous substance;
generating, by a sensing electrode of the sensing component, a current signal between the sensing electrode and a counter electrode of the sensing component indicative of a concentration level of the target gaseous substance in response to a first electrochemical reaction that occurs with respect to the target gaseous substance in an instance in which the sample gaseous substance is incident on the sensing electrode;
maintaining, by a reference electrode of the sensing component, a potential of the sensing electrode at a fixed value; and
modifying, via a bias voltage circuit that is operatively coupled to the sensing electrode, a direction of a second electrochemical reaction that occurs with respect to the interferent gaseous substance.

12. The method of claim 11, wherein the second electrochemical reaction occurs responsive to an above-threshold bias voltage provided to the sensing electrode via the bias voltage circuit.

13. The method of claim 12, wherein the bias voltage circuit has an operating range between 90-150 mV.

14. The method of claim 11, wherein the target gaseous substance comprises Sulfur Dioxide (SO₂).

15. The method of claim 11, wherein the interferent gaseous substance comprises Nitrogen Dioxide (NO₂).
